(19)

Europäisches
Patentamt
European
Patent Office
Office européen
des brevets

(11)　EP 4 523 615 A1

(12)　**EUROPEAN PATENT APPLICATION**

(43) Date of publication:
**19.03.2025  Bulletin 2025/12**

(21) Application number: **23197919.6**

(22) Date of filing: **18.09.2023**

(51) International Patent Classification (IPC):
**A61B 5/026** (2006.01)　　**A61B 5/00** (2006.01)
**A61M 1/06** (2006.01)

(52) Cooperative Patent Classification (CPC):
**A61B 5/4312; A61B 5/0261; A61B 5/4836;
A61M 1/06;** A61B 5/0059

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
 • **GERHARDT, Lutz Christian
   Eindhoven (NL)**
 • **JOHNSON, Mark Thomas
   Eindhoven (NL)**

 • **PAULUSSEN, Elvira Johanna Maria
   Eindhoven (NL)**
 • **VAN LIESHOUT, Ron Martinus Laurentius
   Eindhoven (NL)**
 • **HIWALE, Sujitkumar
   Eindhoven (NL)**
 • **DAMODARAN, Mathivanan
   Eindhoven (NL)**
 • **VERKRUIJSSE, Willem
   Eindhoven (NL)**

(74) Representative: **Philips Intellectual Property &
Standards
High Tech Campus 52
5656 AG Eindhoven (NL)**

(54)　**BLOOD PERFUSION ASSESSMENT OF BREAST**

(57)　Proposed concepts thus aim to provide schemes, solutions, concept, designs, methods and systems pertaining to determining blood perfusion in a subject's breast. In particular, embodiments aim to provide a method for determining blood perfusion in a subject's breast by comparing a blood perfusion parameter to a reference blood perfusion parameter while taking into account a surface pressure (or a parameter indicative/responsive to surface pressure) of a portion of the subject's breast. In this way, a perfusion uniformity value can be determined essentially describing how uniform (i.e., similar) the blood perfusion parameters are to each other.

FIG. 1

EP 4 523 615 A1

**Description**

FIELD OF THE INVENTION

This invention relates to the field of determining blood perfusion in a subject, and more specifically, to the field of determining blood perfusion in a subject's breast.

BACKGROUND OF THE INVENTION

The best source of nutrition for a baby is human milk given by a breastfeeding mother or in the form of expressed milk (e.g. after pumping). One reason why is that the milk contains antibodies which help protect against many common childhood and future illnesses. The WHO recommends exclusively breastfeeding for at least 6 months, meaning no other foods or liquids are provided. Breastfeeding related issues like pain and trauma are among the main causes of weaning from breastfeeding.

In particular, acute nipple pain as a result of nipple or areola vasospasm is associated with early cessation of breastfeeding and comorbidities such as depression, anxiety, and mastitis. Vasospasm is a reduced blood flow through capillaries/arterioles caused by prolonged constriction and/or excessive constriction of the peripheral circulation, resulting in blanching of the nipple. In extreme cases, and if not counteracted or prevented in time, vasospasm of the arterioles causes intermittent ischemia, which is manifested as pallor, followed by cyanosis as the venous blood is deoxygenated and then erythema when reflex vasodilation occurs.

About 25% of first-time mothers report acute nipple pain and burning sensation due to vasospasm. Means to prevent or detect early signs of vasoconstriction are therefore highly desirable for mums as persistent nipple pain is associated with cessation of breastfeeding and comorbidities (depression, anxiety, and mastitis). Typically, vasospasms are only treated once they have occurred.

SUMMARY OF THE INVENTION

The invention is defined by the claims.

According to examples in accordance with an aspect of the invention, there is provided a method for determining blood perfusion in a subject's breast.

The method comprises: obtaining a pressure parameter responsive to a surface pressure of a first portion of a subject's breast; obtaining a blood perfusion parameter of a second portion of the subject's breast; comparing the blood perfusion parameter to a reference blood perfusion parameter; and determining a perfusion uniformity value based on the comparison and the pressure parameter.

Proposed concepts thus aim to provide schemes, solutions, concept, designs, methods and systems pertaining to determining blood perfusion in a subject's breast. In particular, embodiments aim to provide a method for determining blood perfusion in a subject's breast by comparing a blood perfusion parameter to a reference blood perfusion parameter while taking into account a surface pressure (or a parameter indicative of (responsive to) a surface pressure) of a portion of the subject's breast. In this way, a perfusion uniformity value can be determined essentially describing how uniform (i.e., similar) the blood perfusion parameters are to each other.

In other words, it is proposed that by comparing a blood perfusion parameter to a reference blood perfusion parameter while taking into account a surface pressure of a portion of the subject's breast (for instance, whether the blood perfusion parameters were measured at a time point or period of relative maximum or minimum surface pressure), a uniformity of the blood perfusion can be determined. For instance, it can be determined whether at two different times (i.e., points or periods) of minimum surface pressure, the respective blood perfusion parameters of the same breast portion are substantially different to one another, i.e., not uniform. The perfusion uniformity value can then be used as a metrology signature or a clinical decision metric to detect precursors or early signs of vascular changes in general, and vasospasm, in particular.

Alternatively, the reference blood perfusion parameter and the blood perfusion parameter could be measured at the same time but in different portions of the subject's breast. The perfusion uniformity value can then be understood as a uniformity of the blood perfusion in different portions of the subject's breast. This can then be used as an indicator of vasospasm, i.e., if one portion of the subject's breast has much lower blood perfusion than another portion, then this may indicate that the first portion has vasospasm.

The inventors have realised that based on detecting early changes in blood perfusion in a subject's breast (a precursor to vasospasm or very mild vasospasm), timely feedback may be provided to the mother or the operation of a breast pump may be automatically adjusted. Further, by taking into account a surface pressure on a portion of the subject's breast, the comparisons between different blood perfusion measurements can be made more reliable. This surface pressure could arise, for example, from a breast pump being pushed against the breast via the use of a vacuum, or it could arise from a nipple shield being pushed against the breast due to a sucking infant.

Accordingly, this invention may be of particular use in breast pumps or nipple shields, such that a uniformity of blood perfusion can be automatically determined during milk expression such that the mother may be warned if the blood perfusion is too un-uniform, i.e., indicating vasospasm. The operation of a breast pump may also be automatically adjusted in response to the uniformity of blood perfusion.

Ultimately, an improved method for determining blood perfusion in a subject's breast is provided.

In some embodiments, obtaining a blood perfusion parameter may comprise measuring the blood perfusion parameter in the second portion of the subject's breast, wherein the blood perfusion parameter is measured at a time of minimum or maximum surface pressure. Time points or periods of minimum and/or maximum surface pressure provide a more reliable and/or informative time to measure blood perfusion, such that blood perfusion of the breast can be analysed under no or low strain (displacement or relative motion) or relative maximum strain (displacement or relative motion). Further, if a breast pump is being used to administer the surface pressure, the breast pump will stay at minimum or maximum pressure for an extended stationary period (of low / practically-zero relative tissue displacement / motion) and therefore blood perfusion can be measured between cycles in an easier, more effective, and/or more reliable manner.

In some embodiments, the method may further comprise measuring a reference blood perfusion parameter in the second portion of the subject's breast, wherein the reference blood perfusion parameter is measured at a different time of minimum or maximum surface pressure respectively. For instance, if the blood perfusion parameter is measured at a time of minimum surface pressure then the reference blood perfusion parameter will also be measured at a time of minimum surface pressure, however, at a different time, i.e., before or after. The parameters can then be more reliably and/or usefully compared to one another to determine a perfusion uniformity value. Correspondingly, if the blood perfusion parameter is measured at a time of maximum surface pressure, then the reference blood perfusion will also be measured at a time of different maximum surface pressure so that the parameters can be more reliably and/or usefully compared to one another.

In some embodiments, the method may further comprise measuring a reference blood perfusion parameter in a third portion of the subject's breast, wherein the reference blood perfusion is measured at the same time of minimum or maximum surface pressure respectively, and wherein the third portion is separate to the second portion. In this way, at the same time of minimum or maximum surface pressure, the blood perfusion in two different portions of the subject's breast can be measured and compared to one another in order to determine a perfusion uniformity value.

In some embodiments, the method may further comprise generating a control signal based on the perfusion uniformity value. For instance, if the perfusion uniformity value indicates that the perfusion parameters are substantially different to one another (e.g. indicating potential early onset of vasospasm), then a device may be controlled accordingly. For instance, the subject's smartphone may be controlled to alert the subject of this finding or a breast pump being used may be controlled in such a way as to attempt to reduce the non-uniformity of the blood perfusion.

In some embodiments, the control signal may comprise a control instruction configured for modifying a control parameter of a breast pump.

In some embodiments, the control parameter may comprise at least one of: a pumping frequency; a pumping amplitude; a pumping duty cycle; an operational status of the breast pump; and user feedback. These are all control parameters that could be adjusted in response to the perfusion uniformity value in such a way as to attempt to benefit the subject.

In some embodiments, the method may further comprise measuring a calibration blood perfusion parameter describing a difference in blood perfusion between a time of minimum surface pressure and a consecutive time of maximum surface pressure; and wherein determining the perfusion uniformity value is further based on the calibration blood perfusion parameter. In this way, the method may be calibrated, taking into account a 'user-standard' difference in blood perfusion at different surface pressures, such that the perfusion uniformity value may be more reliably determined. For instance, the consecutive times of minimum and maximum surface pressure may be the first times of minimum and maximum surface pressure respectively during a milk-expression / breastfeeding session.

In some embodiments, the perfusion uniformity value may describe a uniformity in the blood perfusion parameter and the reference blood perfusion parameter relative to the pressure parameter. In other words, the perfusion uniformity value may be a value that describes, relative to the pressure parameter (e.g., for the same pressure parameter), how similar/uniform the blood perfusion parameter and the reference blood perfusion parameter are to one another. This may take the form of an absolute difference between the two, a percentage difference between the two, a ratio, and/or any other suitable form of value.

In some embodiments, obtaining a pressure parameter may comprise applying a time-varying surface pressure to the first portion of a subject's breast using a breast pump including a vacuum generator; and determining a pressure parameter responsive to the applied time-varying surface pressure. For example, the rim of a breast pump shield may be pushed against the subject's breast in a time-varying way via the use of a time-varying vacuum. In this case, the first portion of the subject's breast would then be the portion of the breast in contact with the breast pump shield.

In some embodiments, the applied time-varying surface pressure may comprise periodically varying surface pressure. In this way, the times of maximum and minimum surface pressure can be reliably known and therefore, in some embodiments, used to reliably measure the blood perfusion parameters at times of maximum and/or minimum surface pressure.

In some embodiments, the first portion of the subject's breast may comprise the second portion of the subject's breast. In this way, the blood perfusion of the portion which is under pressure can be obtained and the perfusion uniformity value determined for this portion. This portion is a portion at risk of vasospasm during milk expression.

In some embodiments, the second portion of the subject's breast may be separate from the first portion of the subject's breast. In this way, portions of the breast other than that which is under pressure may be monitored for a uniformity of blood perfusion. Portions other than that which is under pressure may also be at risk of vasospasm during milk expression, or used to assess early changes in blood perfusion or vascular change precursor signatures at locations further away from typical locations where vasospasm can occur due to the application of the vacuum or surface pressure.

According to another aspect of the invention, there is provided a computer program comprising code means for implementing the method of any herein disclosed method when said program is run on a processing system.

According to another aspect of the invention, there is provided a system for determining blood perfusion in a subject's breast. The system comprising: an input interface configured to: obtain a pressure parameter responsive to a surface pressure of a first portion of a subject's breast; and obtain a blood perfusion parameter of a second portion of the subject's breast; and a processor configured to: compare the blood perfusion parameter to a reference blood perfusion parameter; and determine a perfusion uniformity value based on the comparison and the pressure parameter.

In some embodiments, the system may further comprise a breast pump including a vacuum generator and configured to apply a time-varying surface pressure to the first portion of the subject's breast; and wherein obtaining the pressure parameter comprises determining the pressure parameter responsive to the applied time-varying surface pressure.

Thus, there may be proposed concepts for determining blood perfusion in a subject's breast, and this may be done based on comparing blood perfusion parameters while taking into account a pressure parameter responsive to a surface pressure of a portion of the subject's breast.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified flow diagram of a method for determining blood perfusion in a subject's breast according to a proposed embodiment;
Fig. 2A is a simplified illustration of a cross-section of a breast pump on a subject's breast;
Fig. 2B is a simplified illustration of how a vacuum affects the fit of a breast pump to a subject's breast;
Fig. 3A is a graph showing an example of blood perfusion values in a portion of a subject's breast corresponding to periodic surface pressure applied to a different portion of the subject's breast;
Fig. 3B is a graph showing an example of blood perfusion values in a portion of a subject's breast corresponding to periodic surface pressure applied to the same portion of the subject's breast;
Fig. 4 is a flow diagram of a method for determining blood perfusion in a subject's breast according to a proposed embodiment;
Fig. 5 is a simplified illustration of a light guide technology configuration for integration into a breast shield;
Fig. 6 is a flow diagram of a method for determining blood perfusion in a subject's breast according to a proposed embodiment;
Fig. 7 is a block diagram of a system for determining blood perfusion in a subject's breast according to a proposed embodiment;
Fig. 8 is a block diagram of a system for determining blood perfusion in a subject's breast including a breast pump according to a proposed embodiment; and
Fig. 9 illustrates an example of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to determining blood perfusion in a subject's breast. According to proposed concepts, a number of possible solutions may be implemented separately or jointly. That is, although these possible solutions may be described below separately, two or more of these possible solutions may be implemented in one combination or another.

Embodiments of the invention aim to provide a method for determining blood perfusion in a subject's breast. This can be achieved by comparing blood perfusion parameters while taking into account a pressure parameter responsive to a surface pressure of a portion of the subject's breast.

Proposed concepts thus aim to provide schemes, solutions, concept, designs, methods and systems pertaining to determining blood perfusion in a subject's breast (e.g., determining a perfusion uniformity value in a subject's breast). In particular, embodiments aim to provide a method for determining blood perfusion in a subject's breast by comparing a blood perfusion parameter to a reference blood perfusion parameter while taking into account a surface pressure (or a parameter indicative/responsive to surface pressure) of a portion of the subject's breast. In this way, a perfusion uniformity value can be determined essentially describing how uniform (i.e., similar) the blood perfusion parameters are to each other.

In other words, it is proposed that by comparing a blood perfusion parameter to a reference blood perfusion parameter while taking into account a surface pressure of a portion of the subject's breast (for instance, whether the blood perfusion parameters were measured at a time of relative maximum or minimum surface pressure), a uniformity of the blood perfusion can be determined. For instance, it can be determined whether at two different times of a longitudinal measurement of minimum surface pressure, the respective blood perfusion parameters are substantially different to one another, i.e., not uniform. The perfusion uniformity value can then be used as a clinical decision metric to detect early signs of vascular changes in general, and vasospasm, in particular.

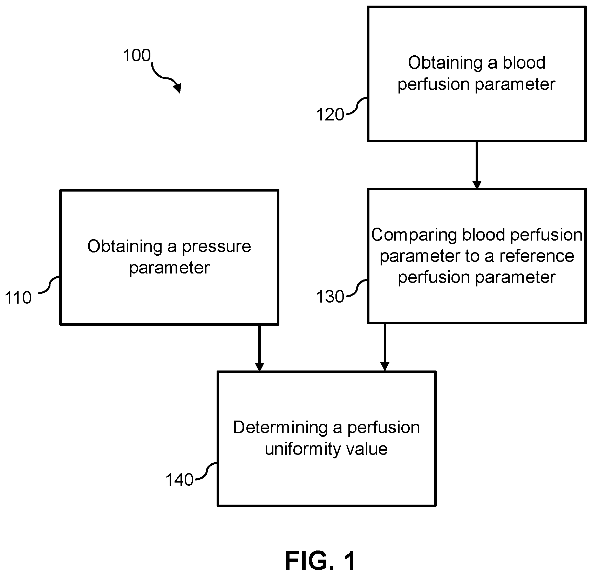

Referring now to Fig. 1, there is depicted a simplified flow diagram of a method 100 for determining blood perfusion in a subject's breast according to a proposed embodiment.

The method 100 begins with the step 110 of obtaining a pressure parameter responsive to a surface pressure of a first portion of a subject's breast. Responsive to a surface pressure means that (actual skin) surface pressure can be derived from the pressure parameter or that the pressure parameter can be derived from the surface pressure. In other words, the pressure parameter may be an absolute measurement of the surface pressure or it may be a proxy for the surface pressure which varies in accordance with the surface pressure. In some embodiments, a breast pump value (such as time elapsed since operation started, vacuum level, power state, voltage drawn, frequency profile, etc.) can be used as the pressure parameter as these values are capable of acting as a proxy for the surface pressure of the subject's breast. As mentioned, in some embodiments, the pressure parameter can be an absolute surface pressure measurement and can be measured using a suitable device such as a (mechanical, optical, etc.) pressure or flow sensor or barometric sensor inside a device. This may be useful, for instance, in situations when the surface pressure is from an infant sucking, and a device to measure the surface pressure can be integrated into a nipple shield, for example.

Surface pressure can be understood as direct, physical (local) surface pressure - for instance, the rim of a breast pump cushion pushing against a breast. The application of a (global) vacuum, for example, to a portion of a breast is not what is meant by a surface pressure. In other words, surface pressure can be understood as direct, physical, and positive surface pressure.

In step 120, a blood perfusion parameter of a second portion of the subject's breast is obtained. For instance, the blood perfusion parameter can be a single value in the units of $l/min/mm^3$, $l/min$ or any other suitable units for blood perfusion. In some embodiments, the blood perfusion parameter can be a modulated blood perfusion signal or a modulated blood flow signal (i.e., comprising multiple blood perfusion values). The blood perfusion parameter can be obtained via measurement or via retrieval of a value or values from an external device/system or a memory. In some embodiments, the blood perfusion parameter can be the difference between the minimum and maximum blood perfusion during a certain time-window, such as a single (periodic or cyclic) vacuum / pressure cycle.

In step 130, the blood perfusion parameter is compared to a reference blood perfusion parameter. For instance, the reference blood perfusion parameter can be another, different blood perfusion parameter. In some embodiments, the reference blood perfusion parameter can be a theoretical expected blood perfusion parameter. In some embodiments, the reference blood perfusion parameter can be the difference between the minimum and maximum blood perfusion during a certain (different) time-window, such as a single (different) vacuum / pressure cycle.

In step 140, a perfusion uniformity value is determined based on the comparison and the pressure parameter. For example, if the blood perfusion parameter and the reference blood perfusion parameter were both obtained at (or if the reference blood perfusion parameter was modelled for) the same or similar pressure parameter (surface pressure), then a perfusion uniformity value that essentially describes the comparison can be used. Alternatively, if the blood perfusion parameter and the reference blood perfusion parameter were obtained at (or if the reference blood perfusion parameter was modelled for) different pressure parameters, then a value describing the comparison can be adjusted to account for this in determining the perfusion uniformity value. In other words, in situations in which the perfusion parameters being compared were measured at the same pressure parameter (or time), the perfusion uniformity value can essentially comprise a value describing their comparison (e.g., a ratio or a percentage difference, etc.). However, in situations in which the perfusion parameters being compared were measured at different pressure parameters (or times), then the perfusion uniformity can comprise a value describing their comparison that has been modified or adjusted in some way to account for the difference in their respective pressure parameters.

For instance, in this embodiment, the perfusion uniformity value describes a uniformity in the blood perfusion parameter and the reference blood perfusion parameter relative to the pressure parameter. In other words, the perfusion uniformity value is a value that describes, relative to the pressure parameter (e.g., for the same pressure parameter), how similar/uniform the blood perfusion parameter and the reference blood perfusion parameter are to one another. This can take the form of an absolute difference between the two, a percentage difference between the two, a ratio, a time differential within a known periodic cycle, and/or any other suitable form of value.

In embodiments in which a breast pump with a vacuum generator is responsible for the time-varying pressure, the perfusion uniformity value can take into account its vacuum cycles when comparing the perfusion parameters. This may essentially take the form of time-synchronizing/correlating the blood perfusion parameter and the reference blood perfusion parameter. This is because, in this case, time is responsive to the surface pressure, i.e., can be used as a proxy for the surface pressure as the vacuum of the breast pump cycles between low and high vacuum.

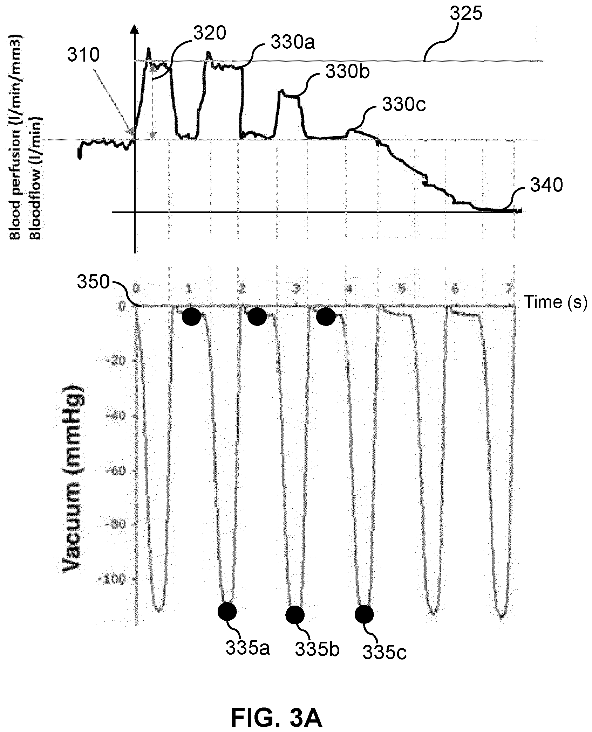

For example, essentially time-synchronized/correlated perfusion or blood flow data with corresponding externally applied pressure parameters (e.g., breast pump vacuum, suction force, etc.) for a well-defined time point or time interval of the pump cycle can be extracted and analyzed. Then, by analyzing said time-correlated data with respect to changes in blood perfusion modulation or flow pattern signatures (e.g., based on altered blood flow or, for example, a 20% perfusion reduction from a perfusion offset baseline, see line 320 in Fig. 3A), an early indication of vasospasm can be determined. A perfusion offset baseline can be understood as the perfusion that is expected at a time of minimum/maximum surface pressure.

In other words, the objective of the present invention can be understood as detecting early signs of vascular changes reflective in blood perfusion or flow parameters (values) such that early signs of vasospasm can be detected and potentially acted on or even prevented. For example, based on detected early vascular changes (precursors to or very mild vasospasm) timely feedback can be given to the mother or a control signal can be generated for controlling the breast pump (e.g., adaptation of pump settings, massage mode, application of heat). This could then potentially fully resolve the precursor changes prior to a severe vasospasm (and/or blanching of the nipple).

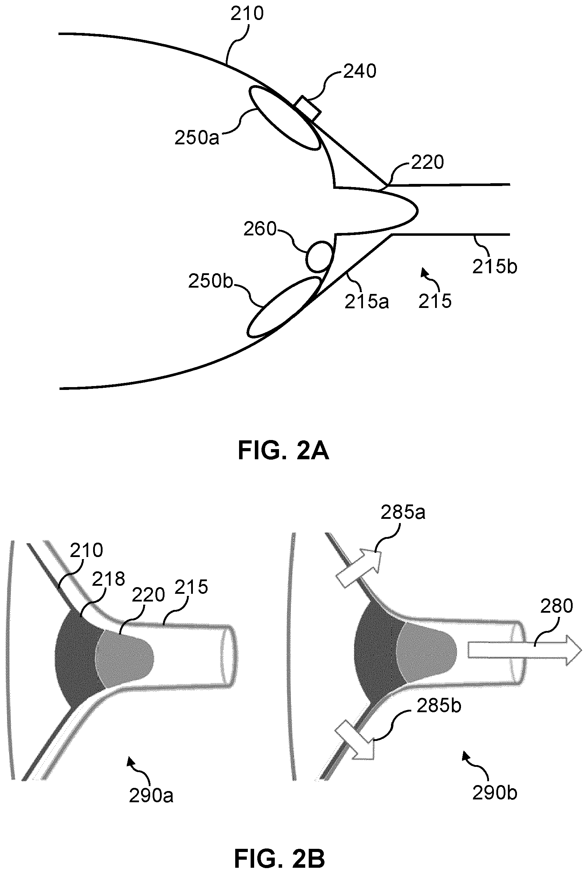

Referring now to Fig. 2A, there is depicted a simplified illustration of a cross-section of a breast pump 215 on a subject's breast 210. The breast pump 215 comprises a breast pump flange 215a and a tube 215b (connecting to the rest of the breast pump, for instance, the vacuum generator). Where the flange 215a meets the subject's breast 210, there is a ring portion of the subject's breast (the cross-sections of which can be seen at 250a and 250b) where the breast will be under surface pressure from the flange 215a pushing against the breast during operation (for instance, as the breast pump forms a vacuum seal with the breast). Portion 260 is provided as an example of a portion of the subject's breast 210 which is not under surface pressure (i.e., separate to the portions, 250a and 250b, which are under surface pressure). The subject's nipple 220 (usually the portion in which clinical vasospasm manifests and is eventually detected) can also be considered a portion of the breast which is separate to those portions, 250a and 250b, which under surface pressure from the rim of the breast pump flange 215a.

Sensor 240 is an example positioning of a sensor for measuring a blood perfusion or flow parameter of a portion 250a of the subject's breast 210 which is under surface pressure. In other words, in this embodiment, the first portion of the subject's breast (the surface pressure of which the pressure parameter describes) comprises the second portion of the subject's breast (for which the blood perfusion parameter is obtained).

The inventors have realized that the blood vessels which terminate at the nipple actually originate at the area of the breast 210 outside the areola (not shown). Therefore, in some embodiments, the inventors propose to particularly monitor the changes in blood perfusion (and, optionally, capillary refill) of the vessels in the area beyond the areole (where the rim / cushion of the flange 215a is only touching the skin and no vacuum is exerted and in action) alone or in addition to vessels actually exposed to the applied vacuum (e.g. inside the open lumen of the breast pump shield 215 where the vacuum is effectively in action).

Referring now to Fig. 2B, there is depicted a simplified illustration of how a vacuum affects the fit of a breast pump (or nipple shield/guard) 215 to a subject's breast 210. As can be seen, the subject's breast 210 comprises the subject's areola 218 and the subject's nipple 220. In state 290a, no vacuum is being applied to the breast pump 215, and the fit can be seen to be loose, i.e., the breast pump 215 is not pressing against or even contacting the subject's breast 210. However, in state 290b, when a vacuum 280 is being applied via the breast pump 215, the subject's breast 210 is pulled into the breast pump and therefore pressed against the breast pump, as is shown by arrows 285a and 285b. Surface pressure is therefore applied to those contacting portions of the breast 210 in the opposite direction to arrows 285a and 285b.

Referring now to Fig. 3A, there is depicted a graph showing an example of blood perfusion values (or a modulated blood flow signal) in a portion of a subject's breast corresponding to periodic surface pressure applied to a different portion of the subject's breast. The x-axis 350 describes time, and at the beginning (i.e., time equals zero) 310, pressure is applied upon which milk expression begins. In this example, pressure is being applied via a vacuum generator in a breast pump and the portion of the subject's breast for which blood perfusion/flow is being measured is a separate portion to the portion to which surface pressure is being applied. Instead, the portion being measured is under the influence of a vacuum (i.e., within the lumen of a breast pump). In other words, the portion of the breast for which blood perfusion/flow is being measured is a portion inside the rim of the flange but not in contact with the rim of the flange (for instance, the nipple).

This graph also clearly shows how the (periodic) vacuum level of a breast pump can be used as a pressure parameter which is responsive to a surface pressure of a first portion of the subject's breast (i.e., the portion which is in contact with the rim of the breast pump flange). For example, as the vacuum level increases, so too will the surface pressure. The vacuum level can thus be used as a proxy for relative surface pressure, i.e., can be used as a pressure parameter acting on a portion of a subject's tissue.

During the first two vacuum cycles, it can be seen that the blood perfusion levels follow substantially the same cycle, i.e., when the vacuum is at a maximum level (and the surface pressure is at a maximum), the blood perfusion values for the first two cycles are substantially the same. In other words, for the first two cycles, the difference between the minimum and maximum blood perfusion 320 is (substantially) the same or very similar. This indicates a uniform blood perfusion, i.e., not indicating vasospasm. In some embodiments of the present invention, the difference between the minimum and maximum blood perfusion 320 (i.e., the difference between the blood perfusion at consecutive times of minimum and maximum surface pressure) can be taken as a calibration blood perfusion parameter. As shown in this example, preferably this calibration blood perfusion parameter is measured in the first cycle of a milk expression session such that the following measurements can be (re-)calibrated. Essentially, because of the applied vacuum, the blood perfusion signal is shifted by a value 320 towards a baseline perfusion offset which depends on the vacuum or suction force applied. This aspect offers opportunity for standardization or a calibration of perfusion measurements under dynamic conditions.

As the milk expression session continues, however, it can be seen that the maximum blood perfusion for subsequent vacuum cycles begins to drop. For example, at consecutive maximum surface pressures at 335a, 335b, and 335c respectively it can be seen that the corresponding blood perfusion values, 330a, 330b, and 330c begin to fall, thereby indicating a non-uniformity of blood perfusion and indicating vasospasm. Full vasospasm can be seen at point 340. In some embodiments, however, after seeing the non-uniformity of blood perfusion parameters between 330a, 330b, and 330c, a control signal or feedback to the user could have been sent to adjust an operation of the breast pump in order to try and prevent the full vasospasm seen at 340. For instance, point 320 or 330a could be taken as the reference blood perfusion parameter and point 330b could be taken as the blood perfusion parameter, and their comparison would show that they are not uniform even though they were obtained at substantially identical vacuum levels (and therefore surface pressures), thereby indicating a precursor of vasospasm. A subsequent comparison then taking point 330b as the reference blood perfusion parameter and point 330c as the blood perfusion parameter would then show the trend continuing.

In other words, in some embodiments, rolling comparisons can be made between subsequent pressure cycles, i.e., the blood perfusion parameter for one comparison can become the reference blood perfusion parameter for the next comparison.

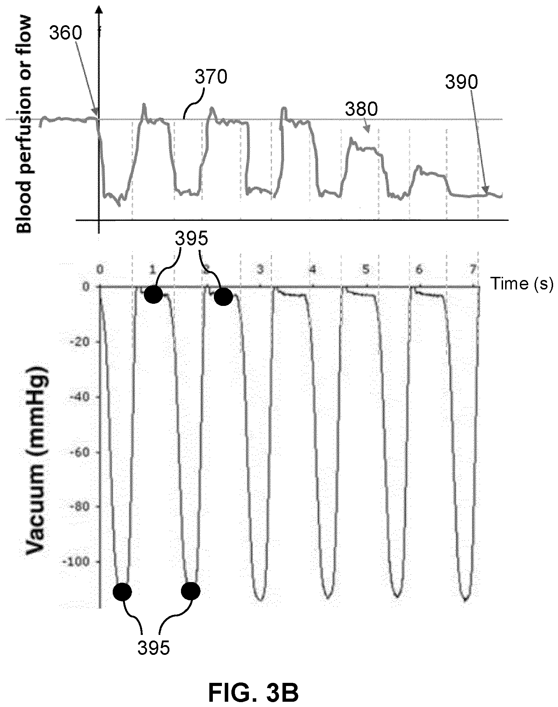

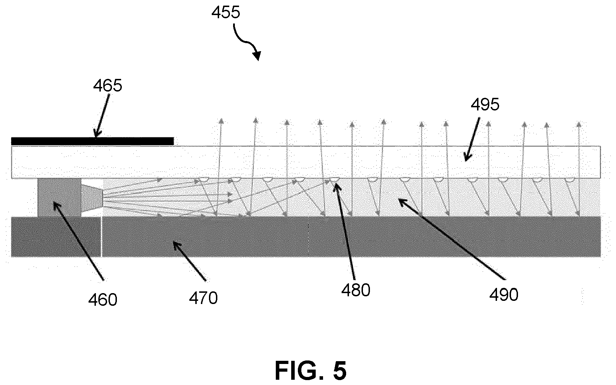

Referring now to Fig. 3B, there is depicted a graph showing an example of blood perfusion values in a portion of a subject's breast corresponding to periodic surface pressure applied to the same portion of the subject's breast (when the vacuum is converted or translated into issue compression as schematically described in Figs. 2A, 2B and embodied in Fig. 5). It can be seen that in this case, the blood perfusion/flow signal is essentially inverted to the one shown in Fig. 3A. This means, as can be seen, that stronger negative vacuum levels (stronger surface pressure or skin compression) correspond to lower perfusion/flow, while lower vacuum levels (or no vacuum) correspond to higher (standard) perfusion.

As the periodic vacuum (surface pressure) begins to be applied 360, the blood perfusion/flow is reduced and leaves its baseline 370. This periodically repeats until onset of vasospasm 380 occurs. Untreated, this vasospasm worsens until full vasospasm 390 occurs. Points 395 are provided as examples of points or periods of zero (or low) breast skin motion, as they are minimum or maximum times of the periodic vacuum (surface pressure) where the slope or time differential of the pressure is zero. These points 395 can also be identified in Fig. 3A (the maximum pressure times labelled as 335a-c while the minimum pressure times are unlabelled but easily recognizable).

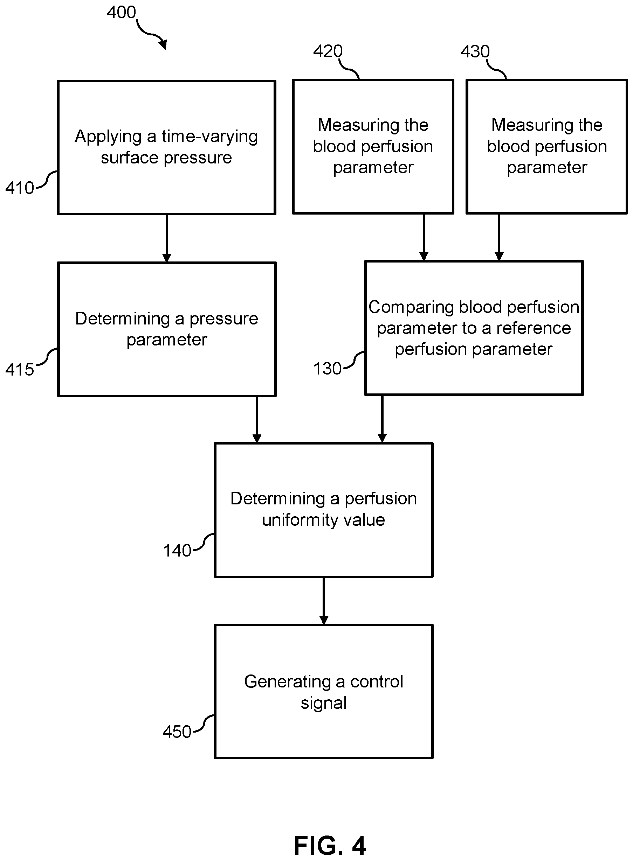

Referring now to Fig. 4, there is depicted a flow diagram of a method 400 for determining blood perfusion in a subject's breast according to a proposed embodiment.

Steps 130 and 140 are substantially the same as have been described in relation to the method 100 in Fig. 1.

In step 410, a time-varying surface pressure is applied to a first portion of a subject's breast using a breast pump including a vacuum generator. For instance, the time-varying surface pressure is applied to the subject's breast via a rim of a flange of the breast pump. For example, as the vacuum in the breast pump increases, the rim of the breast pump flange will push harder against the breast. In this way, a time-varying vacuum in the breast pump will thereby lead to a time-varying surface pressure (i.e. compression) being applied to a portion of the subject's breast. In order to result in beneficial blood perfusion parameters for comparison, the applied surface pressure should be such that it exceeds the pressure needed to cause capillary closure.

In other words, the rim of a breast pump shield can be pushed against the subject's breast in a time-varying way via the use of a time-varying vacuum generator. In this case, the first portion of the subject's breast would be the portion of the breast in contact with the breast pump shield.

In this embodiment, the applied time-varying surface pressure comprises periodically varying surface pressure. In this way, the times of maximum and minimum surface pressure can be reliably known and therefore, in some embodiments, used to reliably measure the blood perfusion parameters at times of maximum and/or minimum surface pressure. In other embodiments, however, the time-varying surface pressure can comprise any suitable varying surface pressure.

In step 415, a pressure parameter is determined responsive to the applied time-varying surface pressure. For instance, a relative pressure parameter can be determined corresponding to the variation of the applied surface pressure (e.g., in response to the variation of the vacuum levels). In other words, a pressure parameter can be determined that, for example, on a scale of 0-100 describes how the applied surface pressure (vacuum level) is varying (e.g., with 0 representing the minimum applied surface pressure, and 100 representing the maximum applied surface pressure). This is merely an example of how the pressure parameter could be determined responsive to the applied surface pressure, however.

In step 420, the blood perfusion parameter in a second portion of the subject's breast is measured, wherein the blood perfusion parameter is measured at a time of minimum or maximum surface pressure. Times of minimum and/or maximum surface pressure (i.e., times of minimum and/or maximum vacuum) provide a more reliable and/or informative time to measure blood perfusion, such that blood perfusion of the breast can be analysed under no strain or relative maximum strain. Further, if a breast pump is being used to administer the surface pressure (usually well above or arteriole capillary closure pressure), the breast pump will stay at minimum and/or maximum pressure for an extended period and therefore blood perfusion can be measured between cycles in an easier, more effective, and/or more reliable manner. Further, by only measuring / imaging at times of minimum or maximum surface pressure, a reduction of measured data is facilitated as the measuring / imaging is done at time of low or zero tissue motion and thus is made easier and less likely to need more data.

Minimum and/or maximum surface pressure can be understood as a temporally local minimum and/or maximum surface pressure respectively, at which the to-be-image skin does not undergo relative motion or temporarily keeps a well-defined strain level. For instance, this can be understood as the minimum and/or maximum surface pressure within one cycle of a periodically varying surface pressure or, alternatively, the minimum and/or maximum surface pressure within a one, two, three, four, five, ten, twenty, or thirty second window respectively. At a time of minimum or maximum surface pressure can also be understood as at a time of minimum or maximum of the pressure parameter.

In this embodiment, the first portion of the subject's breast comprises the second portion of the subject's breast. In this way, the blood perfusion of the portion which is under pressure can be obtained and the perfusion uniformity value determined for this portion. This portion is a portion at risk of vasospasm during milk expression such as a nipple.

For example, in contrast to the changes of blood perfusion in the nipple (which in most cases is not in contact with the surface of the pump, see Figs. 2A and 2B), the time-varying surface pressure (due to the rim of the breast pump flange) with the breast will cause a reduction of blood perfusion in the first/second portions as the pump vacuum increases and thus applies pressure on the tissue, as seen in Fig. 3B. This, however, may also be the base if measuring the perfusion of a nipple that is so elastic that it actually makes contact with the flange of the breast pump funnel. This is also the case with nipple shields /guards when an infant is feeding, wherein the baby sucking is essentially the time-varying vacuum which results in the portions of the nipple shield / guard in contact with portions of the breast exerting a surface pressure on said portions of the breast. This is also the case with suction applied through a Niplette™ syringe for flat or inverted nipples, which through gentle suction pulls the nipple out into a small plastic thimble-like cup.

As vacuum is applied to the nipple, the breast will be pressed against the wall of the pump (resulting in a relatively high surface pressure / pressure parameter). The pressure on the breast tissue will cause blood to be pushed out of the surface capillaries causing a reduction of perfusion. Normally, the tissue will re-perfuse to a baseline as the vacuum is removed and the pressure on the breast in contact with the wall of the pump is removed (i.e., the perfusion will be uniform relative to surface pressure between pressure cycles). If the re-perfusion starts to decrease (i.e., if the perfusion is not uniform relative to surface pressure between pressure cycles) then this could be an early indication that these vessels are not re-perfusing the nipple sufficiently and may lead to a nipple blanching - this would obviously cause pain and need time and/or an intervention to remedy. It would obviously be preferable therefore to prevent vasospasm before it occurs.

In step 430, a reference blood perfusion parameter is measured in the second portion of the subject's breast, wherein the reference blood perfusion parameter is measured at a different time of minimum or maximum surface pressure respectively. For instance, if the blood perfusion parameter is measured at a time of minimum surface pressure then the reference blood perfusion parameter will also be measured at a time of minimum surface pressure, however, at a different time (within the periodic pressure profile). The parameters can then be more reliably and/or usefully compared to one another to determine a perfusion uniformity value. Correspondingly, if the blood perfusion parameter is measured at a time of maximum surface pressure, then the reference blood perfusion will also be measured at a time of different maximum surface pressure so that the parameters can be more reliably and/or usefully compared to one another.

In some embodiments, the (reference) blood perfusion parameter can be measured using LED light guide technology integrated into a breast pump or nipple shield, for example (such as will be described in Fig. 5). In other embodiments, a short-wavelength infrared (SWIR) sensor can be used to measure the blood perfusion parameter. In yet another embodiment, a laser diode with self-mixing interference can be used as a source-sensor system to measure a speckle pattern in order to obtain a (reference) blood perfusion parameter. This might be, for instance, in conjunction with a CMOS (RGB) camera.

In step 450, a control signal is generated based on the perfusion uniformity value (or in other words, responsive to the perfusion uniformity value). For instance, if the perfusion uniformity value indicates that the perfusion parameters are substantially different to one another (e.g. indicating potential vasospasm), then a device can be controlled accordingly. For instance, the subject's smartphone can be controlled to alert the subject of this finding or a breast pump being used can be controlled in such a way as to attempt to reduce the non-uniformity of the blood perfusion.

In other words, in some embodiments, the control signal is for controlling a display device to display the perfusion uniformity value or generated content responsive to the perfusion uniformity value. For example, the control signal could be for controlling a display device (e.g., a smartphone) to tell the subject to use additional vitamin supplements, e.g., vitamin B6 with Niacin, Magnesium, Evening Primrose Oil or even medications such as Nifedipine.

In this embodiment, however, the control signal comprises a control instruction configured for modifying a control parameter of a breast pump, wherein the control parameter comprises at least one of: a pumping frequency; a pumping amplitude; a pumping duty cycle; an operational status of the breast pump; and user feedback. These are all control parameters that could be adjusted in response to the perfusion uniformity value in such a way as to attempt to benefit the subject. For example, an operational status of the breast pump can comprise switching to a massage mode, to start applying heat, or stopping all operations.

In some embodiments, the control signal that is generated is responsive to the exact value (or band of values) of the perfusion uniformity value. For instance, if the value shows that the perfusion is severely non-uniform, then the control signal could be to cease all operation of a breast pump. However, if the value only shows that the perfusion is slightly non-uniform, then the control signal could be, for example, to reduce the intensity of the breast pump's vacuum.

Referring now to Fig. 5, there is depicted a simplified illustration of a light guide technology configuration 455 for integration into a breast shield, for instance, the breast shield of Fig. 2B. For example, the configuration 455 comprises an LED array side-emitting surface mounted device 460; a perimeter mask 465; a reflective LCD assembly 470; a micro-lens array 480 (not visible); a light guide plate 490; and a glass protective cover 495.

In a first example embodiment of a method for determining blood perfusion in a subject's breast, the (reference) blood perfusion parameter (signal modulation) is measured using LED light guide technology that is integrated into the cone flange of a breast-pump, such as that shown in Fig. 5. Due to the vacuum, the breast is pulled into the cone and therefore a surface pressure is applied to the portion of the breast which is in contact with the cone. Due to the applied vacuum, tissue is pushed against the wall of the breast shield and compressed. This can be seen clearly, for example, in Fig. 3B.

This will force blood out of the tissue. By using the cone (breast pump funnel/flange) itself as a light guide with a wavelength that is absorbed by the blood fraction of the tissue, the amount of perfusion of the tissue can be measured. In this embodiment, the blood perfusion signal modulation will be such that at times of low vacuum, the perfusion remains at baseline, and at times of high vacuum (i.e., high surface pressure) the perfusion is relatively lower. In other words, as the vacuum is converted/translated into skin compression. Blood is pushed out and should re-perfuse to a baseline perfusion upon removal of the vacuum (barring any onset of vasospasm).

Because the pressure cycle (frequency profile of the pump) is known, the contact pressure modulation and cycle of the tissue touching the cone (maximized absorption of the light due to high tissue perfusion) is also known (i.e., the pressure cycle is the pressure parameter and this is responsive to the surface pressure of a first portion of the breast). Therefore, when the cone is forced towards the tissue, the blood in said contacting tissue is pushed out which results in a reduction of light absorption. This can be measured and converted into a blood perfusion parameter. Using these two conditions then, it is possible to essentially calculate or determine the tissue perfusion recovery in time, assuming the reduction of tissue perfusion recovery develops during a prolonged pump cycle.

In a second example embodiment, a short-wavelength infrared (SWIR) sensor is used in combination with an IR source, and the time-domain is studied at the pulsatile part of the perfusion. In other words, the perfusion can be derived from a speckle pattern and would be an absolute perfusion within one vacuum cycle or a perfusion averaged over a vacuum (half) cycle to observe slow vasospasm during the pumping process.

Alternatively, instead of a separate source and SWIR sensor, a laser diode with self-mixing interference can be used as a source-sensor system to measure a speckle pattern.

In this example embodiment, the breast tissue that is in contact with the breast pump is measured, in the position where the breast starts to be pushed into the funnel (e.g., at the edge of the areola). This portion of the breast is less sensitive to motion during pumping / feeding and so is a more reliable portion of the breast to measure / image.

In a third example embodiment, in response to analyzing the perfusion of a nipple during use of a breast pump and finding indication of vasospasm, the breast pump frequency is adapted to optimize the tissue perfusion recovery. At the same time, the nipple perfusion is measured. This is an acute marker for nipple vasoconstriction. If the nipple perfusion is decreased, the breast pump can be halted (i.e., extend the zero-skin deformation motion period of pump vacuum profile) and pain relief feedback can be pushed to the subject. Other actions could also be triggered which include the application of heat provided with the breast pump (e.g., via a heating element, radio frequency, near-infrared light, etc.).

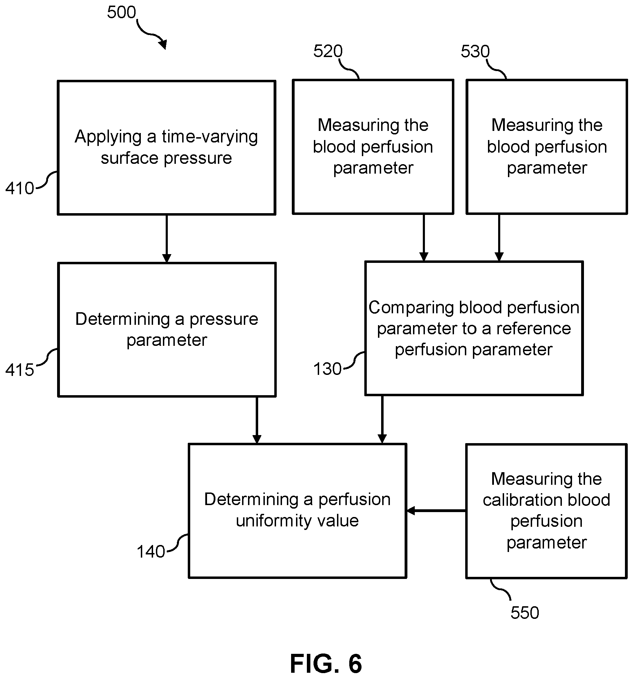

Referring now to Fig. 6, there is depicted a flow diagram of a method 500 for determining blood perfusion in a subject's breast according to a proposed embodiment.

Steps 130 and 140 are substantially the same as have been described in relation to the method 100 in Fig. 1. Steps 410 and 415 are substantially the same as have been described in relation to the method 400 in Fig. 4.

In step 520, the blood perfusion parameter in a second portion of the subject's breast is measured, wherein the blood perfusion parameter is measured at a time of minimum or maximum surface pressure. However, in this embodiment, in contrast to step 420 in method 400, the second portion of the subject's breast is separate from the first portion of the subject's breast. In this way, a portion of the breast other than that which is under pressure can be monitored for a uniformity of blood perfusion. Portions other than that to which pressure is being applied are also at risk of vasospasm during mechanical loading due to breastfeeding and milk expression.

In other words, if the portion / area of the breast being monitored does not contact the wall of a pump (or nipple guard, etc.), but is under the influence of a vacuum (i.e., within the lumen of the breast pump), such as a nipple, then the effect of applying a vacuum (i.e., increasing surface pressure on the separate first portion of the breast) would actually draw more blood into the capillaries in the second portion compared to a baseline perfusion (at zero vacuum). This increased perfusion will usually return to the baseline level (such as 325 in Fig. 3A) when the vacuum (surface pressure) is removed. As a result, in this case, the early sign of vasospasm would actually be a reduction of blood drawn into the capillaries under the influence of a vacuum.

In step 530, a reference blood perfusion parameter in a third portion of the subject's breast is measured, wherein the reference blood perfusion is measured at the same time of minimum or maximum surface pressure respectively, and wherein the third portion is separate to the second portion. In this way, at the same time of minimum or maximum surface pressure (pressure parameter), the blood perfusion in two different portions of the subject's breast can be measured and compared to one another in order to determine a perfusion uniformity value. In other words, this embodiment allows for the blood perfusion of two different portions of the breast to be simultaneously measured and then compared. In other embodiments, however, the blood perfusion of two different portions of the breast can be measured at different but equivalent times of surface pressure and then compared.

In step 550, a calibration blood perfusion parameter is measured describing a difference in blood perfusion between a time of minimum surface pressure and a consecutive time of maximum surface pressure; and wherein determining the perfusion uniformity value is further based on the calibration blood perfusion parameter. In this way, the method 500 can be calibrated, taking into account a 'user-standard' difference in blood perfusion at different surface pressures, such that the perfusion uniformity value can be more reliably determined. For instance, the consecutive times of minimum and maximum surface pressure can be the first times of minimum and maximum surface pressure respectively during a milk-expression / breastfeeding session.

For example, in this embodiment, step 140 of determining the perfusion uniformity value involves taking into account the calibration blood perfusion parameter such that the determined perfusion uniformity value can be made more reliable. For instance, if both the reference blood perfusion parameter and the blood perfusion parameter describe a perfusion that is far from that which the calibration blood perfusion parameter indicates as normal, then this can indicate that the readings are not reliable or, alternatively, that both parameters are indicating vasospasm.

In a fourth example embodiment, a one wavelength laser speckle contrast (LASCA) system comprising a laser diode (e.g., 650nm) and a CMOS (RGB) camera can be used to measure a portion of the breast which is not in contact with the breast pump or nipple shield. In this instance, said portion is the subject's nipple. For instance, the laser diode and CMOS camera can be positioned on the portion of the breast pump or nipple shield into which the nipple will enter but, ideally, not touch the sides. They can therefore be configured to measure the blood perfusion (or flow) of the nipple during milk expression, and preferably only during still moments when the breast is stable and not-moving (i.e., at minimum or maximum times of surface pressure (or vacuum) where the slope or time differential of the pressure is zero such as points 395 in Fig. 3B). This could be, for instance, between intervals of a baby sucking on a nipple or at times of minimum or maximum vacuum. It should be noted that a lens (e.g., a cylindrical lens or axicon) or a diffuser can be added to the laser diode to avoid having a point source.

Both a single image snapshot or a sequence of images corresponding to a no or low skin-motion interval are suitable. If measurements are only taken at still moments (i.e., with low or zero relative motion between skin and breast pump device), this will greatly reduce the image data volume to analyze - this is therefore done in this example embodiment.

In this example embodiment, a breast pump with a vacuum generator is being used. Therefore, in this embodiment, it is possible to measure the blood perfusion at ambient vacuum level, where the nipple has a distance from the breast pump funnel. This is the time where the breast is most stable. The blood perfusion is also measured at maximum vacuum level, where the breast is also stable as the skin or the nipple experiences a short time where the skin does not move relatively to the pump.

A blood perfusion signal for this example embodiment could look like that shown in Fig. 3A. An onset of vasospasm would then be obvious from either a step function of the modulated signal or a decrease in the modulation amplitude (i.e., the difference between the minimum and maximum blood perfusion during a single vacuum cycle).

A key aspect of this example embodiment is to start monitoring blood flow/perfusion after inducing a controlled complete capillary/arteriole closure event (pressure: 15-35 mmHg) in order to detect early signs of vasospasm characterized by altered blood flow/perfusion signature (amplitude, loss of modulation). A crucial and inventive feature is the time correlation / synchronization of the perfusion imaging with the breast pump pressure cycle, wherein the blood perfusion is imaged during pressure levels both above and below capillary closure pressure levels. This therefore provides an optimal differentiation between normal blood vessels and vasospasm, especially if the skin or the to-be-imaged blood vessels are subjected to (e.g., cyclic, period) mechanical loading during the perfusion measurement. Essentially, in this example embodiment, the blood perfusion parameter (and the reference blood perfusion parameter) is the difference between the minimum and maximum blood perfusion during a single pressure cycle (e.g., value 320 in Fig. 3A). These differences can therefore be compared to one another in order to determine a perfusion uniformity value.

As mentioned, in this example embodiment, measurements (images) should be taken at pressure periods both above and below capillary closure levels, resulting in an image period between two consecutive closure events. The capillary closure event (stimulus) is used as a reference proper baseline for reliable longitudinal measurements (within the same breastfeeding / pumping sessions or during the course of successive sessions). Essentially, a capillary closure event early in a milk expression session can be used as a calibration blood perfusion parameter.

A capillary closure event can be considered a mechanical stimulus capable of being induced by: the application of (breast pump) device pressure levels above capillary closure levels; any general tissue deformation exceeding capillary closure pressure; and/or a critical pressure-time threshold exerted on a nipple from a device or infant.

In a fifth example embodiment, the difference in blood perfusion between breast portions outside and inside a vacuum cup (breast shield/cone/flange) is measured (e.g., between a portion at the edge of the nipple and a portion at the edge of the areola). The nipple-areola complex (NAC) receives its blood supply from three main arteries (internal thoracic, lateral thoracic, and posterior intercostal arteries) through a number of smaller branches. Any external pressure on these smaller branches can lead to reduced blood supply to NAC. This can happen when an external device such as a breast pump is tightly placed over NAC and negative pressure is applied for suction. The resulting reduced blood supply can lead to hypoperfusion in NAC.

There are two possible ways to measure the blood flow reduction inside the cone/shield area of a breast pump. The area inside the cone/shield is a first possible area to measure blood perfusion using different imaging configurations and sensors, as previously elaborated. Based on empirical data or a pre-determined historical value, it is thus possible to determine a certain threshold which indicates significant blood flow reduction.

Another way is to use the comparative analysis of an area inside the cone to an area outside the cone to find a comparative reduction in the perfusion in the area inside the cone. Based on such comparative analysis, certain thresholds can be determined which indicate significant blood flow reduction in the area inside the cone and thus a high probability of hypoperfusion. Such a differential measurement allows one to distinguish perfusion change due to vasospasm from perfusion change due to applying a vacuum level. The response time of perfusion to vacuum changes should be fast and measurable.

For a particular type or design of a breast pump cone, the reduction in blood flow or perfusion, $BF_{Reduction}$, in area A (an area inside the cone/shield) should be directly related to (negative) pressure applied by the cone of the breast pump on superficial vessels, which would in turn be related to the pressure used for suction, $S_{Pressure}$, and its duration, $S_{Duration}$. In mathematical form, this can be expressed as:

$$BF_{Reduction} = f(S_{Pressure}, S_{Duration}) \qquad (1)$$

The hypoperfusion in NAC would thus be directly related to reduced blood flow to NAC area. Therefore:

$$NAC_{Hypoperfusion} = f(BF_{Reduction}) \hspace{3cm} (2)$$

Using empirical data and experimentation, it is therefore possible to determine thresholds or limits of suction pressure and its duration for a particular type of breast pump cone, which can result in NAC hypoperfusion for an individual or a group of lactating mothers. This information can then be used for early prediction of hypoperfusion.

In a sixth example embodiment, a first perfusion offset signal amplitude or gradient (i.e., slope/leading edge) can be used for perfusion calibration as a function of (sucking) force. As seen in Fig. 3A, the blood perfusion signal in a non-contact portion of the breast is shifted by a value 320 towards a baseline perfusion offset that depends on the vacuum or suction force applied. This value 320 or a slope thereof can then be used for calibration purposes, particularly if random, non-periodic forces are applied.

The value 320 is likely not a constant but more an offset with an AC modulation, the amplitude of which depends upon the pumping rate (perfusion response times are generally a couple of seconds). The initial perfusion increase should scale with the sucking force and thus facilitate a method for calibration in this situation. As perfusion is increased in response to the vacuum applied, a second measurement (e.g., with a single point sensor like a photodiode) could be added on the edge of the vacuum area (but where the pump still exerts mechanical force on the breast) to do a calibration measurement (for instance, of the value 320).

Depending on the prevailing loading condition and type of imaging (contact or non-contact), the initial perfusion signal can be an offset perfusion (e.g., in Fig. 3A in the case where vacuum is applied to the portion being measured) or a reperfusion signal (e.g., in Fig. 3B in the case where surface pressure is applied to the portion being measured) from blood flowing back into an unloaded tissue area (e.g., after compression or the biting of an infant on the nipple).

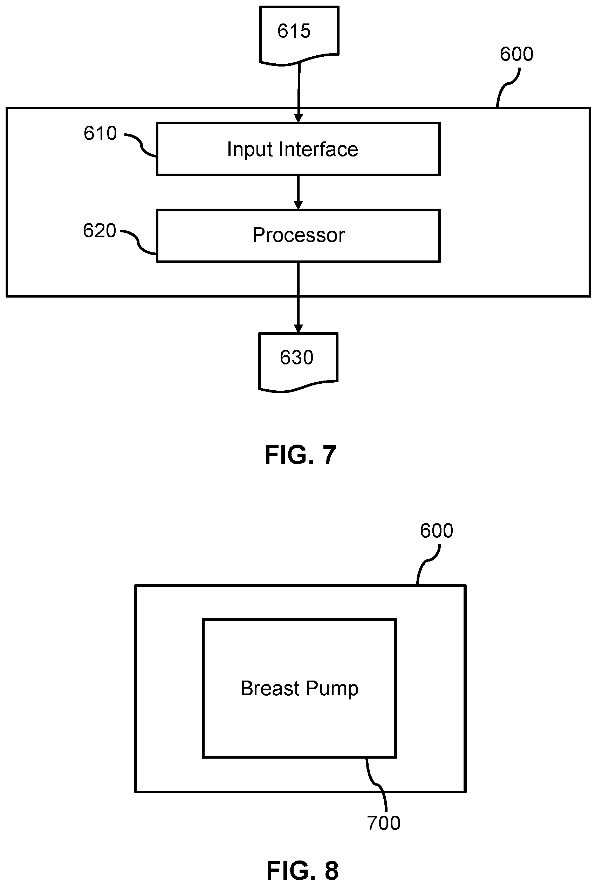

Referring now to Fig. 7, there is depicted a system 600 for determining blood perfusion in a subject's breast according to a proposed embodiment. The system 600 comprises an input interface 610 and a processor 620.

The system 600 is configured determine a perfusion uniformity value by processing inputs 615. The inputs 615 comprise a pressure parameter responsive to a surface pressure of a first portion of a subject's breast and a blood perfusion parameter of a second portion of the subject's breast. The input interface 610 obtains the inputs 615 and the processor 620 is then configured to compare the blood perfusion parameter to a reference blood perfusion parameter and then determine an output 630. The output 630 comprises a perfusion uniformity value based on (i.e. responsive to) the comparison and the pressure parameter.

Referring now to Fig. 8, there is depicted a block diagram of a system 600 for determining blood perfusion in a subject's breast including a breast pump 700 according to a proposed embodiment. Essentially, the system 600 is the same as the system 600 depicted in Fig. 7, however, with the addition of a breast pump 700. The breast pump 700 includes a vacuum generator and is configured to apply a time-varying surface pressure to the first portion of the subject's breast.

In other embodiments, the system 600 can comprise a nipple guard/shield, or a Niplette™ device for inverted nipple, instead of a breast pump 700.

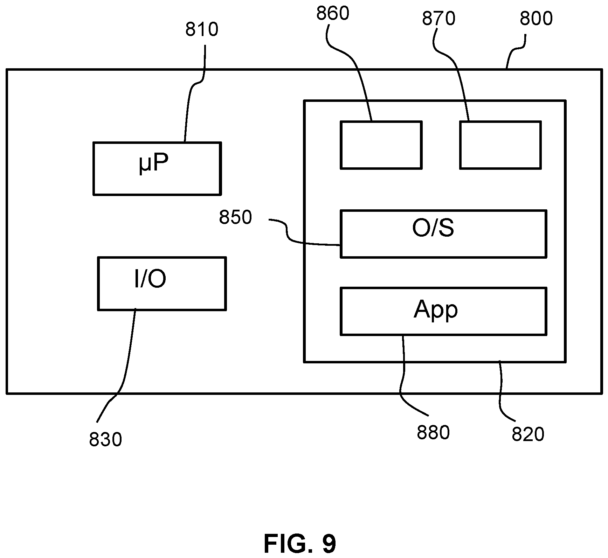

Fig. 9 illustrates an example of a computer 800 within which one or more parts of an embodiment may be employed. Various operations discussed above may utilize the capabilities of the computer 800. In this regard, it is to be understood that system functional blocks can run on a single computer or may be distributed over several computers and locations (e.g. connected via internet).

The computer 800 includes, but is not limited to, PCs, workstations, laptops, PDAs, palm devices, servers, storages, and the like. Generally, in terms of hardware architecture, the computer 800 may include one or more processors 810, memory 820 and one or more I/O devices 830 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 860, source code 870, and one or more applications 880 in accordance with exemplary embodiments. As illustrated, the application 880 comprises numerous functional components for implementing the features and operations of the exemplary embodiments. The application 880 of the computer 800 may represent various applications, computational units, logic, functional units, processes, operations, virtual entities, and/or modules in accordance with exemplary embodiments, but the application 880 is not meant to be a limitation.

The operating system 850 controls the execution of other computer programs, and provides scheduling, input-output control, file and data management, memory management, and communication control and related services. It is contemplated by the inventors that the application 880 for implementing exemplary embodiments may be applicable on all commercially available operating systems.

Application 880 may be a source program, executable program (object code), script, or any other entity comprising a set of instructions to be performed. When a source program, then the program is usually translated via a compiler (such as the compiler 860), assembler, interpreter, or the like, which may or may not be included within the memory 820, so as to operate properly in connection with the O/S 850. Furthermore, the application 880 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 830 may include input devices such as, for example but not limited to, a mouse, keyboard, scanner, microphone, camera, etc. Furthermore, the I/O devices 830 may also include output devices, for example but not limited to a printer, display, etc. Finally, the I/O devices 830 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 830 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at start-up, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 880 and the O/S 850 are read, in whole or in part, by the processor 810, perhaps buffered within the processor 810, and then executed.

When the application 880 is implemented in software it should be noted that the application 880 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 880 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1,4 and 6, and the systems of Figs. 7 and 8, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 9 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

**Claims**

1. A method (100) for determining blood perfusion in a subject's breast, the method comprising:

   obtaining a pressure parameter (110) responsive to a surface pressure of a first portion of a subject's breast;
   obtaining a blood perfusion parameter (120) of a second portion of the subject's breast;
   comparing the blood perfusion parameter (130) to a reference blood perfusion parameter; and
   determining a perfusion uniformity value (140) based on the comparison and the pressure parameter.

2. The method of claim 1, wherein obtaining a blood perfusion parameter comprises:
   measuring the blood perfusion parameter (420) in the second portion of the subject's breast, wherein the blood perfusion parameter is measured at a time of minimum or maximum surface pressure.

3. The method of claim 2, further comprising:
   measuring a reference blood perfusion parameter (430) in the second portion of the subject's breast, wherein the reference blood perfusion parameter is measured at a different time of minimum or maximum surface pressure respectively.

4. The method of claim 2, further comprising:
   measuring a reference blood perfusion parameter (530) in a third portion of the subject's breast, wherein the reference blood perfusion is measured at the same time of minimum or maximum surface pressure respectively, and wherein the third portion is separate to the second portion.

5. The method of any prior claim, further comprising generating a control signal (450) based on the perfusion uniformity value.

6. The method of claim 5, wherein the control signal comprises a control instruction configured for modifying a control parameter of a breast pump, and preferably, wherein the control parameter comprises at least one of: a pumping frequency; a pumping amplitude; a pumping duty cycle; an operational status of the breast pump; and user feedback.

7. The method of any prior claim, further comprising:

    measuring a calibration blood perfusion parameter (550) describing a difference in blood perfusion between a time of minimum surface pressure and a consecutive time of maximum surface pressure; and
    wherein determining the perfusion uniformity value is further based on the calibration blood perfusion parameter.

8. The method of any prior claim, wherein the perfusion uniformity value describes a uniformity in the blood perfusion parameter and the reference blood perfusion parameter relative to the pressure parameter.

9. The method of any prior claim, wherein obtaining a pressure parameter comprises:

    applying a time-varying surface pressure (410) to the first portion of a subject's breast using a breast pump including a vacuum generator; and
    determining a pressure parameter (415) responsive to the applied time-varying surface pressure.

10. The method of claim 9, wherein the applied time-varying surface pressure comprises periodically varying surface pressure.

11. The method of any prior claim, wherein the first portion of the subject's breast comprises the second portion of the subject's breast.

12. The method of any of claims 1 to 10, wherein the second portion of the subject's breast is separate from the first portion of the subject's breast.

13. A computer program comprising code means for implementing the method of any preceding claim when said program is run on a processing system.

14. A system (600) for determining blood perfusion in a subject's breast, the system comprising:

    an input interface (610) configured to:

        obtain a pressure parameter responsive to a surface pressure of a first portion of a subject's breast; and
        obtain a blood perfusion parameter of a second portion of the subject's breast; and

    a processor (620) configured to:
    compare the blood perfusion parameter to a reference blood perfusion parameter; and
    determine a perfusion uniformity value based on the comparison and the pressure parameter.

15. The system of claim 14, wherein the system further comprises:

    a breast pump (700) including a vacuum generator and configured to:
    apply a time-varying surface pressure to the first portion of the subject's breast; and
    wherein obtaining the pressure parameter comprises:
    determining the pressure parameter responsive to the applied time-varying surface pressure.

FIG. 1

FIG. 2A

FIG. 2B

FIG. 3A

FIG. 3B

FIG. 4

**FIG. 5**

500

520

530

Applying a time-varying
surface pressure

410

Measuring the
blood perfusion
parameter

Measuring the
blood perfusion
parameter

Determining a pressure
parameter

415

Comparing blood perfusion
parameter to a reference
perfusion parameter

130

Determining a perfusion
uniformity value

140

Measuring the
calibration blood
perfusion
parameter

550

**FIG. 6**

615

600

610 — Input Interface

620 — Processor

630

**FIG. 7**

600

Breast Pump

700

**FIG. 8**

810

860    870    800

μP

850 — O/S

I/O

App

830

880    820

**FIG. 9**

Europäisches
Patentamt

European
Patent Office

Office européen
des brevets

# EUROPEAN SEARCH REPORT

**Application Number**

EP 23 19 7919

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/154382 A1 (BARTLETT RUSH [US] ET AL) 27 May 2021 (2021-05-27) * the whole document * | 1,5,6, 8-15 | INV. A61B5/026 A61B5/00 A61M1/06 |
| X | MIRIAM VAN DER HOEK ET AL: "Cutaneous perfusion of the human lactating breast: a pilot study with laser Doppler perfusion monitoring", PHYSIOLOGICAL MEASUREMENT, INSTITUTE OF PHYSICS PUBLISHING, BRISTOL, GB, vol. 40, no. 5, 3 June 2019 (2019-06-03), XP020336420, ISSN: 0967-3334, DOI: 10.1088/1361-6579/AB1AD7 [retrieved on 2019-06-03] * the whole document * | 1,14,15 | |
| A | CN 108 498 086 A (SHENZHEN RUIBO TECH CO LTD) 7 September 2018 (2018-09-07) * the whole document * | 1-15 | |
| A | US 2007/219449 A1 (WATMOUGH DAVID J [GB]) 20 September 2007 (2007-09-20) * paragraphs [0016] - [0017] * * paragraph [0074] * * claims 1-2 * | 1-15 | TECHNICAL FIELDS SEARCHED (IPC) A61B A61M |
| A | CN 116 546 953 A (AVILA THERAPEUTICS INC) 4 August 2023 (2023-08-04) * abstract; figure 4 * | 1-15 | |

The present search report has been drawn up for all claims

| Place of search | Date of completion of the search | Examiner |
|---|---|---|
| The Hague | 6 February 2024 | Loveniers, Kris |

EPO FORM 1503 03.82 (P04C01)

## ANNEX TO THE EUROPEAN SEARCH REPORT
## ON EUROPEAN PATENT APPLICATION NO.

EP 23 19 7919

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2024

| Patent document cited in search report | | | Publication date | Patent family member(s) | | | Publication date |
|---|---|---|---|---|---|---|---|
| US 2021154382 | A1 | | 27-05-2021 | US | 2020078503 | A1 | 12-03-2020 |
| | | | | US | 2021154382 | A1 | 27-05-2021 |
| | | | | WO | 2020051438 | A1 | 12-03-2020 |
| CN 108498086 | A | | 07-09-2018 | NONE | | | |
| US 2007219449 | A1 | | 20-09-2007 | EP | 1750575 | A1 | 14-02-2007 |
| | | | | US | 2007219449 | A1 | 20-09-2007 |
| | | | | WO | 2005107577 | A1 | 17-11-2005 |
| CN 116546953 | A | | 04-08-2023 | NONE | | | |

EPO FORM P0459

For more details about this annex : see Official Journal of the European Patent Office, No. 12/82